# EUROPEAN PATENT APPLICATION

(11) **EP 2 679 153 A1**
(43) Date of publication of application: **01.01.2014**
(21) Application number: 12173587.2
(22) Date of filing: 26.06.2012
(51) Int. Cl.: A61B 5/11, H01H 3/14

(54) **Induction pad**

(71) Applicant: China Medical University, Taichung City 404 (TW)
(72) Inventor: Chiou, Jin-Chern, 404 Taichung City (TW); Lo, Shih-Che, 404 Taichung City (TW); Tsai, Hsin-Hsueh, 404 Taichung City (TW); Yan, Jia-Hung, 700 Tainan City (TW); Chang, Fong Yuan, 408 Taichung City (TW); Chang, Hui-Mei, 402 Taichung City (TW)
(74) Representative: 2K Patentanwälte Blasberg Kewitz & Reichel

(57) **Abstract**

An induction pad includes a first induction layer (10), a second induction layer (20) and a spacer layer (30). The second induction layer (20) has at least one second sparse induction zone (21) and at least one second dense induction zone (22). The spacer layer (30) is disposed between the first induction layer (10) and second induction layer (20), and includes at least one high pressure spacer zone (31) and at least one low pressure spacer zone (32). The second induction layer (20) is pressed downwards upon receiving a load to compress the spacer layer (30) and contact the first induction layer (10) to generate electric connection, thereby detecting the pressed location. Through the dense induction zone (22) and sparse induction zone (21) distributed on the second induction layer (20) whether a person is lay on a bed can be judged to reduce faulty judgments, and the posture of the person can be detected to better understand conditions of the person lay on the bed.

## Description

### FIELD OF THE INVENTION

The present invention relates to an induction pad and particularly to an induction pad equipped with contact or weight sensors.

### BACKGROUND OF THE INVENTION

To many patients and aged people bed is necessary for rest and rehabilitation. Hence many nursing institutions have installed monitoring cameras to monitor whether the patients or aged people have rested on the beds timely to maintain good health and also to understand their sleeping quality and conditions. However, such a scheme makes the people under monitoring uncomfortable mentally and cannot rest easily and also feel intrusion of privacy.

With advances of medical and technology medical equipments have a great deal of improvements in recent years. Now some beds can be equipped with weight sensors on the bed board with four weighing means installed on four legs of the bed, then by calculating through a formula the gravity center location of the person lay on the bed can judge whether the person has left the bed or changed the posture. Such an approach free the bedridden people the uncomfortable feeling of being watched by the monitoring cameras. But the aforesaid technique of judging the posture alteration via deduction of change of the gravity center through a formula could result in a greater range of faulty judgment. It also provides insufficient information in terms of the positional conditions of the person lay on the bed.

### SUMMARY OF THE INVENTION

The primary object of the present invention is to solve the problems of greater range of faulty judgment and insufficient information of positional conditions of the person lay on the bed that occurs to the conventional bed mattress equipped with weight sensors.

To achieve the foregoing object the present invention provides an induction pad which includes a first induction layer, a second induction layer and a spacer layer. The second induction layer has at least one second sparse induction zone and at least one second dense induction zone. The spacer layer is disposed between the first induction layer and second induction layer, and includes at least one high pressure spacer zone and at least one low pressure spacer zone. The second induction layer is pressed downwards upon receiving a load to compress the spacer layer and contact the first induction layer to generate electric connection, thereby detect the pressed location.

Thus, by distributing the at least one dense induction zone and the at least one sparse induction zone of the second induction layer, the dense induction zone can detect a smaller area while the spare induction zone can detect a larger area, and through the characteristics of weight contact area of objects, desirable induction zones can be arranged to make detection in an effective range, thereby to judge whether a person is lay on a bed and also detect person's posture on the bed to reduce faulty judgment and better understand person's conditions in using the bed.

The foregoing, as well as additional objects, features and advantages of the invention will be more readily apparent from the following detailed description, which proceeds with reference to the accompanying drawings.

### BRIEF DESCRIPTION OF THE DRAWINGS

- FIG. 1: is a cross section of a first embodiment of the invention.
- FIG. 2: is a schematic view of an arrangement layout of induction zones and spacer zones of the first embodiment.
- FIG. 3: is a schematic view of an arrangement layout of the induction zones of the first embodiment.
- FIG. 4: is a schematic view of the structure an induction member of a second embodiment of the invention.
- FIG. 5: is a cross section of the spacer structure of a third embodiment of the invention.
- FIG. 6: is a schematic view of the spacer structure of a fourth embodiment of the invention.
- FIG. 7: is a schematic view of apertures distribution of the second induction layer of a fifth embodiment.
- FIG. 8: is a schematic view of the spacer structure of a sixth embodiment of the invention.
- FIG. 9: is a schematic view of induction zone distribution of a seventh embodiment of the invention.
- FIG. 10: is a schematic view of the induction pad of an eighth embodiment of the invention incorporating with a sickbed.

### DETAILED DESCRIPTION OF THE PREFERRED EMBODIMENTS

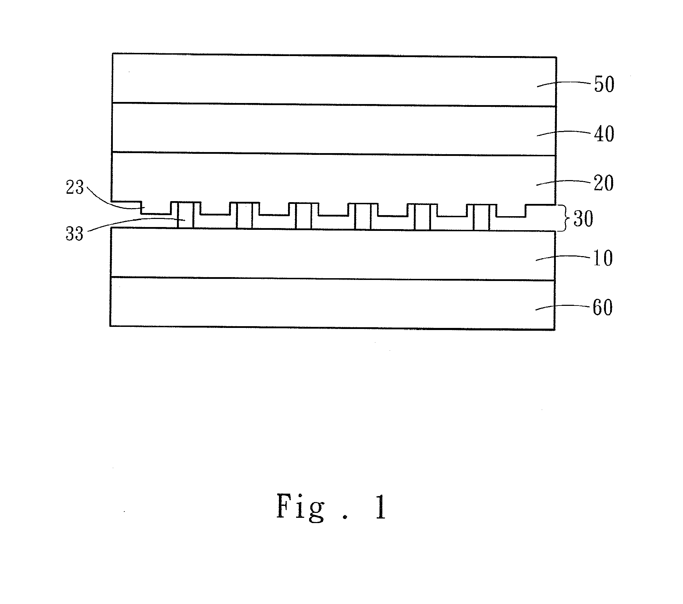

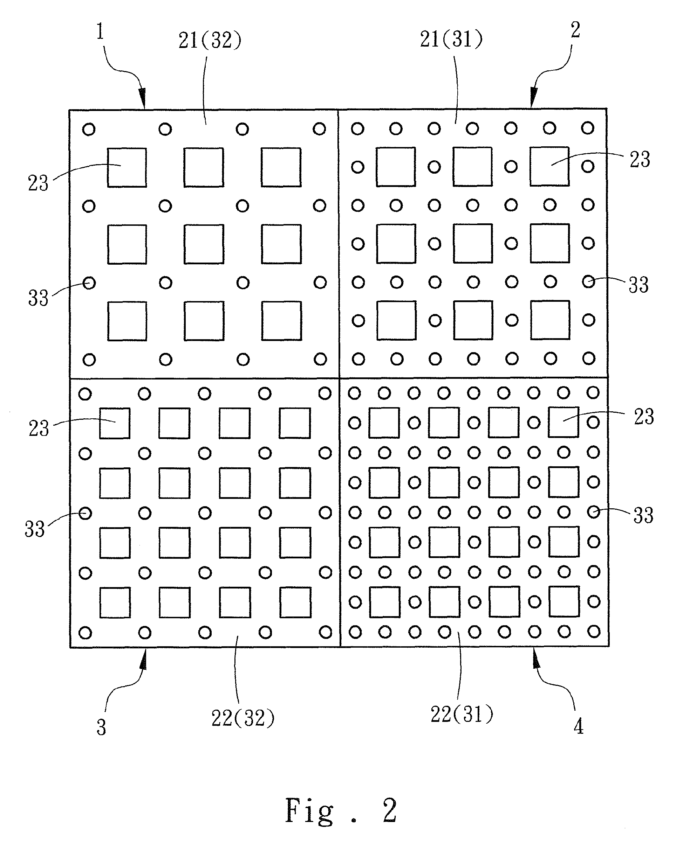

Please refer to FIGS. 1 and 2 for a first embodiment of the induction pad of the invention. The induction pad includes a first induction layer 10, a second induction layer 20 and a spacer layer 30. The second induction layer 20 has at least one second sparse induction zone 21 and at least one second dense induction zone 22. The spacer layer 30 is disposed between the first induction layer 10 and second induction layer 20, and includes at least one high pressure spacer zone 31 and at least one low pressure spacer zone 32. The second induction layer 20 has a plurality of induction members 23 located thereon that can be pressed downwards upon receiving a load to compress the spacer layer 30 and contact the first induction layer 10 to generate electric connection, thereby detect the pressed location.

This embodiment further includes a pliable layer 40 overlapping with the second induction layer 20 so that the second induction layer 20 is interposed between the pliable layer 40 and spacer layer 30 to make person lay on the bed to feel more comfortable. Through the pliable layer 40, the load can be applied indirectly to the second induction layer 20 to detect the location of the person. In addition, a waterproof and air permeable layer 50 may also be added and overlapped with the pliable layer 40 to make the pliable layer 40 interposed between the water and air permeable layer 50 and second induction layer 20, so that a bedridden person who does not toss around frequently on the bed can be free from inflicting decubitus due to sweltering, and the pliable layer 40 also does not need to be washed frequently for persons who have bed wetting problem. In the event that the induction pad of the invention is lay on a bedstead which is not a flat board, a rigid layer 60 is added overlapping with the first induction layer 10 which is interposed between the rigid layer 60 and spacer layer 30. Thereby the second and first induction layers 20 and 10 can bear the load of the person lay thereon to accurately detect the weighing locations.

Also referring to FIG. 2, the induction members 23 are lay denser in the second dense induction zone 22 than in the second sparse induction zone 21, thus the second dense induction zone 22 has a greater induction resolution than the second sparse induction zone 21 and is more desirable to detect a smaller contact area. The high pressure spacer zone 31 has a plurality of spacers 33 located thereon more densely than in the low pressure spacer zone 32, thus can withstand a greater pressure to prevent damage of the induction members 23 between the second induction layer 20 and first induction layer 10 caused by too heavy of load. Hence the high pressure spacer zone 31 is preferably to be positioned on a heavier loading position. By arranging the second sparse induction zone 21 and second dense induction zone 22 incorporated with the high pressure spacer zone 31 and low pressure spacer zone 32, four types of induction detection zones with different characteristics are generated as follow:
A induction zone 1: The second sparse induction zone 21 incorporating with the low pressure spacer zone 32 desirable to detect a larger contact area and a lighter load;
B induction zone 2: The second sparse induction zone 21 incorporating with the high pressure spacer zone 31 desirable to detect a larger contact area and a greater load;
C induction zone 3: The second dense induction zone 22 incorporating with the low pressure spacer zone 32 desirable to detect a smaller contact area and a lighter load; and
D induction zone 4: The second dense induction zone 22 incorporating with the high pressure spacer zone 31 desirable to detect a smaller contact area and a greater load.

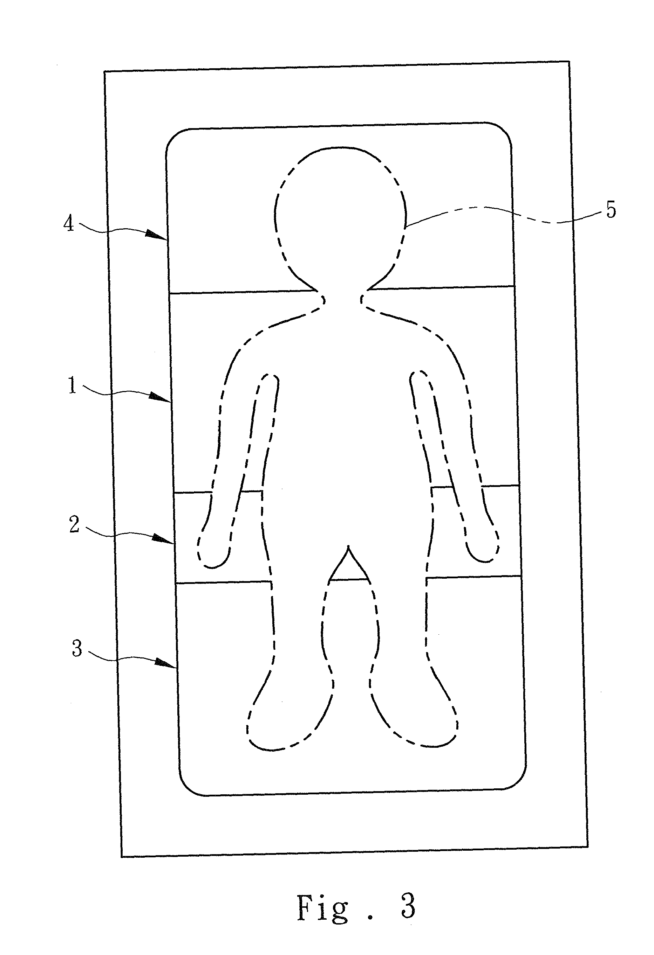

Please refer to FIG. 3 for a layout of the induction zones of the first embodiment. In this embodiment the induction zones are arranged according to a human body 5, from an upper side to a lower side, into a head zone, a back and waist zone, a buttock zone and a leg zone. The head zone requires a smaller induction area and bears a concentrated load, hence is mated with a D induction zone 4. The back and waist zone requires a larger induction area with distributed load, hence is mated with an A induction zone 1. The buttock zone is the gravity center of the human body and bears the greatest load at a larger area, hence is mated with a B induction zone 2; and the leg zone requires a smaller induction area and a smaller load, hence is mated with a C induction zone 3. Thus, the induction zones of the induction pad can be arranged according to the load and area characteristics of the human body 5 to avoid damages of the induction members 23 caused by too heavy of pressure. Moreover, the second sparse induction zone 21 can reduce the number of the induction members 23 needed while providing detection in an effective range.

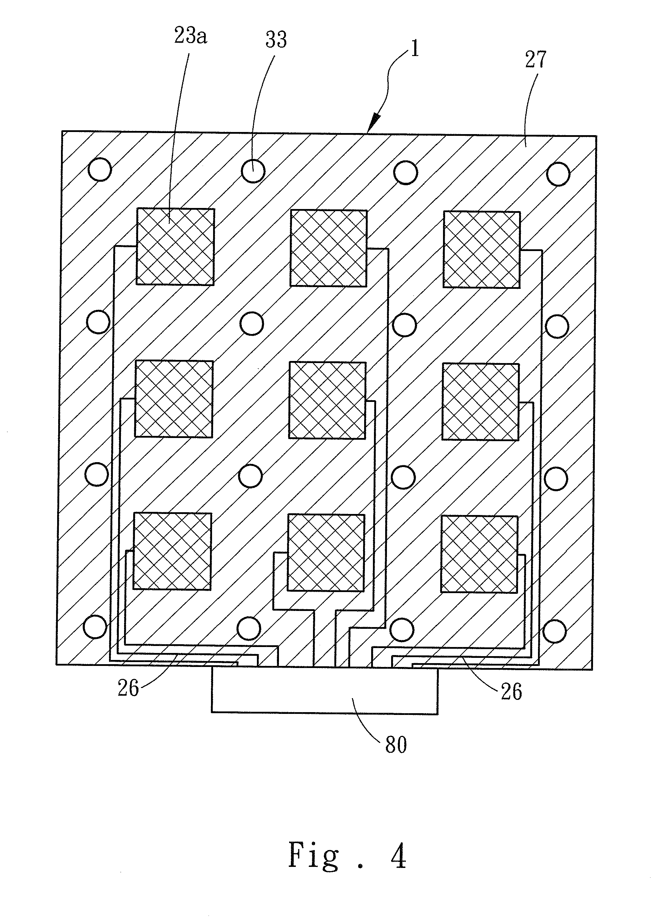

Please refer to FIG. 4 for a second embodiment of the invention. In this embodiment the induction members 23a are designed in a lattice structure to reduce the number of the induction members 23a needed without diminishing the induction capability. Each induction member 23a is connected to a detection unit 80 via a second conductive wire 26. The detection unit 80 detects whether the first induction layer 10 is contact with the second induction layer 20 through conductive connection of the induction member 23a, then judge the contact location of user's load on the second induction layer 20. Moreover, the second conductive wire 26 has an insulation layer 27 to prevent conductive connection between the second conductive wire 26 and first induction layer 10 when the first induction layer 10 is overlapped with the second induction layer 20 to avoid faulty judgment of the detection unit 80.

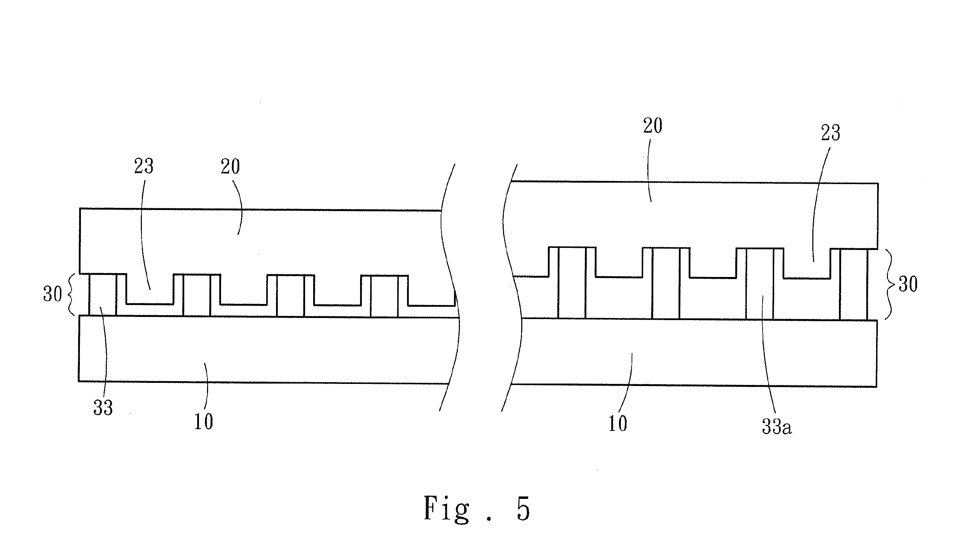

Please refer to FIG. 5 for a third embodiment of the invention. Aside from including denser spacers 33 in the high pressure spacer zone 31, other spacers 33a formed at a greater thickness also are provided to increase the contact interval of the second induction layer 20 and first induction layer 10, thereby prevent damage of the induction members 23 caused by over contact due to bearing too heavy of load lay on the second induction layer 20 and first induction layer 10.

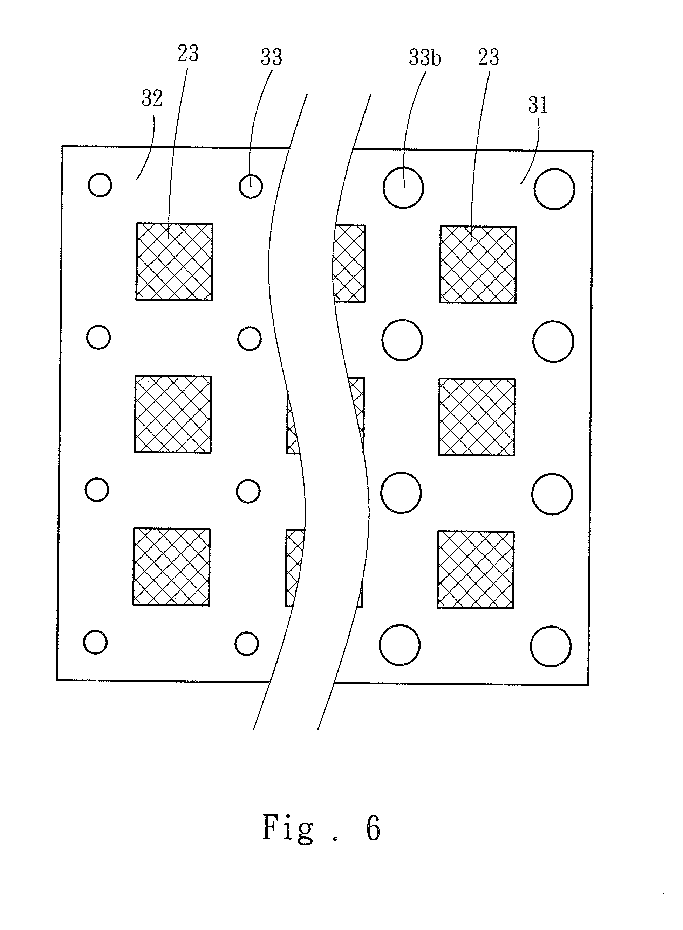

Please refer to FIG. 6 for a fourth embodiment of the invention. The spacers 33b located in the high pressure spacer zone 31 have a greater contact area than the spacers 33 located in the low pressure spacer zone 32. The contact area means the contact area of the spacers 33 and 33b with the first and second induction layers 10 and 20. Such an arrangement allows the spacers 33b in the high pressure spacer zone 31 to bear a greater load, and a greater weight has to be loaded on the high pressure spacer zone 31 to generate induction for the induction members 23 located in high pressure spacer zone 31. Moreover, the spacers 33b in the high pressure spacer zone 31 may also be made from material of a greater hardness to achieve the same effect mentioned above by loading a greater weight to generate the induction for the induction members 23. In short, by adjusting the distribution density, contact area, thickness and material of the spacers 33, the pressure bearing capability of the corresponding spacer zone can be changed; moreover, the sensitivity in response to the pressure of the induction members 23 in the spacer zone can also be adjusted indirectly.

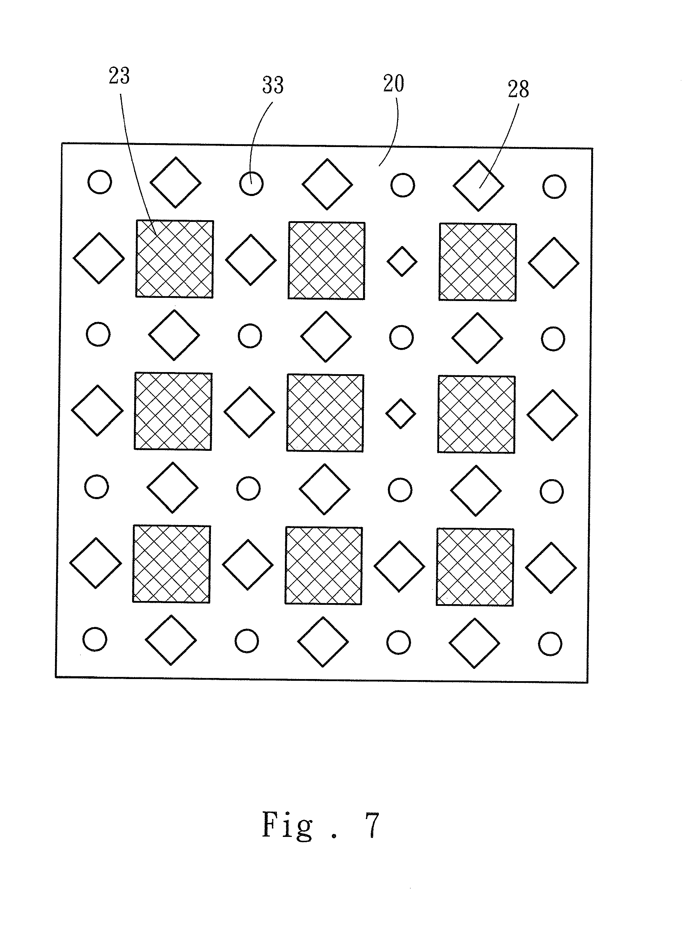

Please refer to FIG. 7 for a fifth embodiment of the invention. In this embodiment the second induction layer 20 has a plurality of apertures 28 distributed among the induction members 23 without affecting operation thereof. The apertures 28 can enhance air permeability to improve comfort of the person lay on the pad.

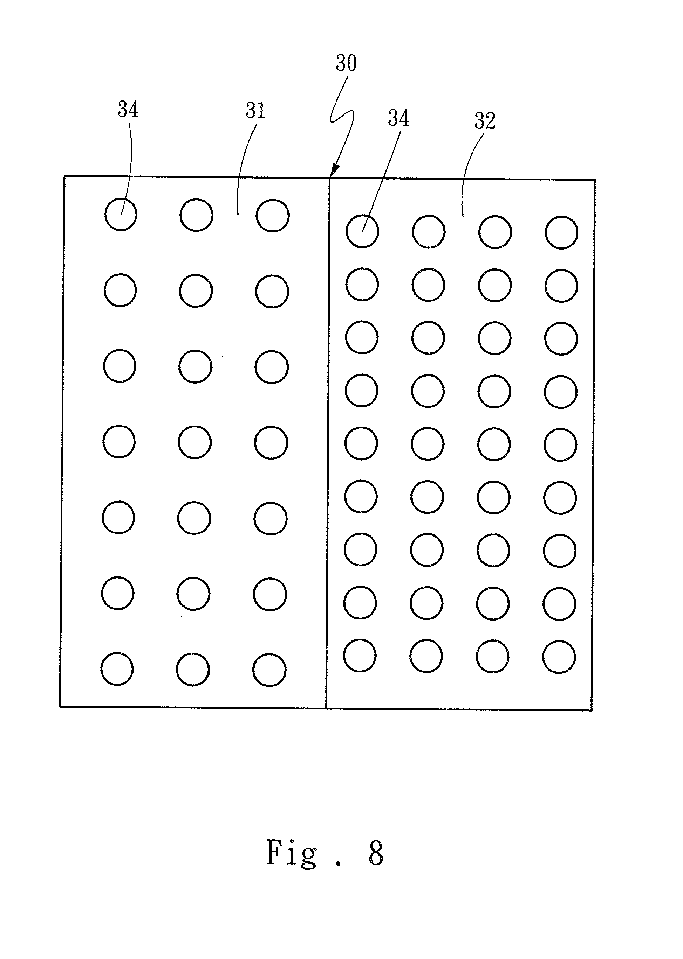

Please refer to FIG. 8 for a sixth embodiment of the invention. In this embodiment the spacer layer 30 is a sheet with a plurality of holes 34 formed thereon. When the second induction layer 20 is pressed downwards upon receiving a load, it contacts the first induction layer 10 through the holes 34 to form electric connection to detect the press location. The holes 34 in the high pressure spacer zone 31 is distributed sparser than that in the low pressure zone 32, thus the high pressure spacer zone 31 can bear a greater load, while the second induction layer 20 in the low pressure zone 32 has a higher sensitivity.

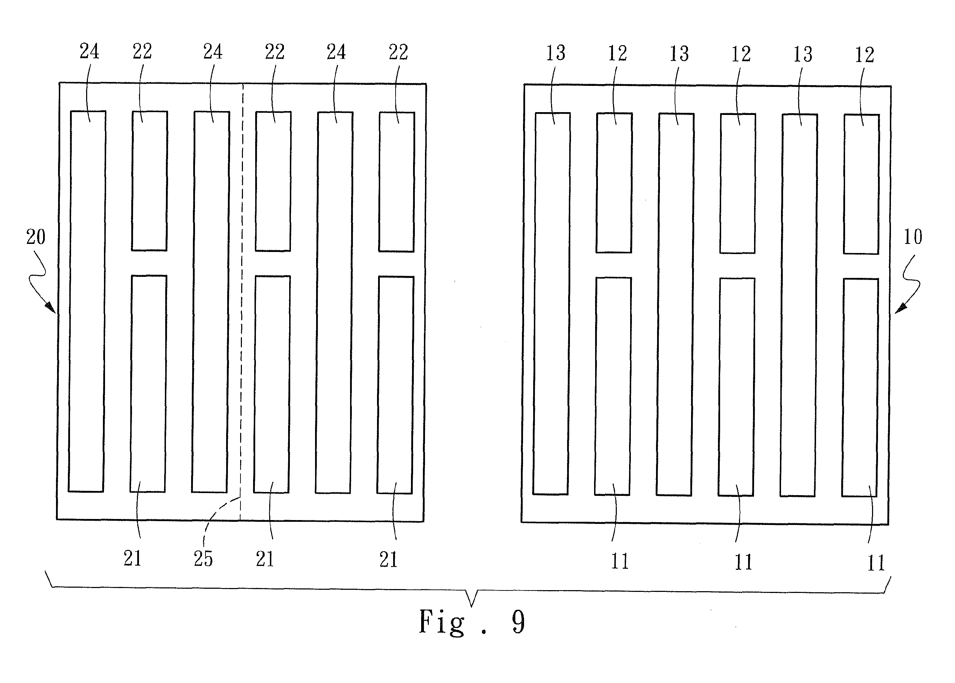

Please refer to FIG. 9 for a seventh embodiment of the invention. In this embodiment the second induction layer 20 further includes at least one second corresponding zone 24, and the first induction layer 10 includes at least one first sparse induction zone 11, at least one first dense induction zone 12 and at least one first corresponding zone 13. The first sparse induction zone 11 and first dense induction zone 12 are overlapped with the second corresponding zone 24, and the second sparse induction zone 21 and second dense induction zone 22 are overlapped with the first corresponding zone 13 so that they are connected to form electric connection upon receiving a load. Through the second sparse induction zone 21 and second dense induction zone 22, the contact location can be judged. Similarly, the second corresponding zone 24 contacts the first spare induction zone 11 and first dense induction zone 12 to form electric connection when a load is applied thereon, thereby can judge the load contact location via the first spare induction zone 11 and first dense induction zone 12.

Therefore, on the second induction layer 20 the second corresponding zone 24 can be designed in a symmetrical manner against the second sparse induction zone 21 and second dense induction zone 22 on the left and right sides through a center axis 25 to make a mold based on the second induction layer 20 as a model to fabricate two sheets of induction layers with the sparse induction zone, dense induction zone and corresponding zones of the same locations, one sheet is for the first induction layer 10 and another sheet for the second induction layer 20. The two induction layers can be overlapped with each other in a up and down manner with the first sparse induction zone 11 and first dense induction zone 12 mating and overlapping with the second corresponding zone 24, and the second sparse induction zone 21 and second dense induction zone 22 mating and overlapping with the first corresponding zone 13. Hence only set of mold is needed. Production cost is lower, and the sparse and dense induction zones can be provided and configured as desired.

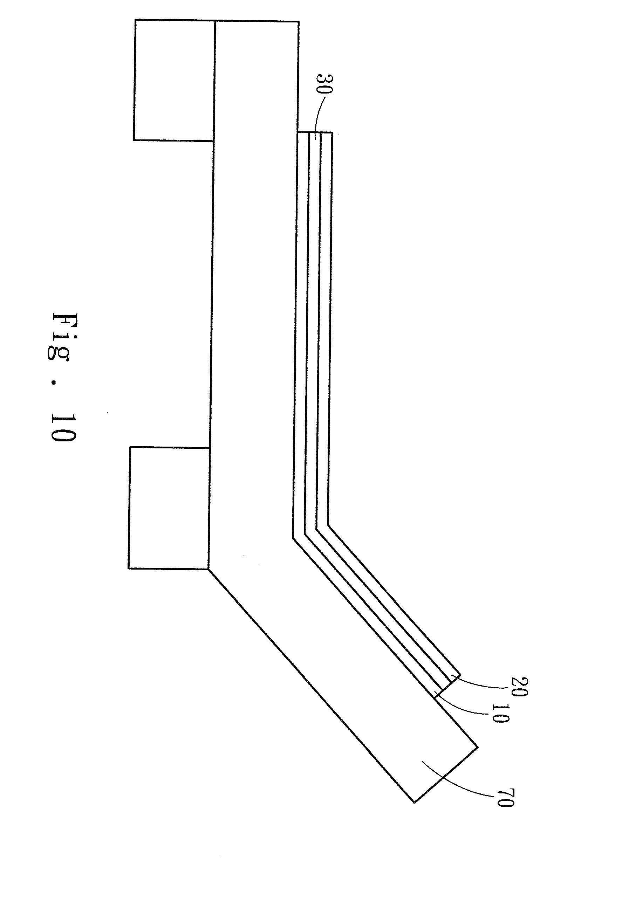

Please refer to FIG. 10 for an eighth embodiment of the invention. Due to a general sickbed 70 now in use can be raised upwards to support a patient to sit up and lift the upper half of body, the first induction layer 10, second induction layer 20 and spacer layer 30 can also be made from flexible material to mate the upward folding movement of the sickbed without being damaged by bending.

In summary an induction pad is disclosed. The induction pad includes a first induction layer 10, a second induction layer 20 and a spacer layer 30. The second induction layer 20 has at least one second sparse induction zone 21 and at least one second dense induction zone 22. The spacer layer 30 is disposed between the first induction layer 10 and second induction layer 20, and includes at least one high pressure spacer zone 31 and at least one low pressure spacer zone 32. The second induction layer 20 is pressed downwards upon receiving a load to compress the spacer layer 30 and contact the first induction layer 10 to generate electric connection, thereby detecting the pressed location. Through the dense induction zone 22 and sparse induction zone 21 distributed on the second induction layer 20 whether a person is lay on a bed can be judged to reduce faulty judgments, and the posture of the person can be detected to better understand conditions of the person lay on the bed.

As a conclusion, the invention, through the sparse induction zone, dense induction zone, high pressure spacer zone and low pressure spacer zone, can generate four different types of induction zones to mate an object which induction is needed. The invention, not only can judge whether a person is lay on a bed, also can detect the posture of the person on the bed, and reduce faulty judgment and better understand use conditions of the person on the bed. By providing a pliable layer, waterproof and air permeable layer and rigid layer, in addition to the inherent flexibility, it can meet use requirements of the sickbed. By providing a symmetrical structure about the center axis in the induction zone of the induction layer, two sets of induction layers of the same structure can be made to overlap with each other in a up and down manner via only one set of mold to provide a layout with a sparse induction zone and a dense induction zone to save production cost.

## Claims

1. An induction pad, **characterized by** the induction pad comprising:
a first induction layer (10);
a second induction layer (20); and
a spacer layer (30) disposed between the first induction layer (10) and the second induction layer (20), the spacer layer (30) including at least one high pressure spacer zone (31) and at least one low pressure spacer zone (32), the second induction layer (20) being pressed downwards upon receiving a load to compress the spacer layer (30) and contact the first induction layer (10) to form electric connection.

2. The induction pad of claim 1, wherein the second induction layer (20) includes at least one second sparse induction zone (21) and at least one dense induction zone (22).

3. The induction pad of claim 2, wherein the second induction layer (20) further includes at least one second corresponding zone (24), the first induction layer (10) including at least one first sparse induction zone (11), at least one first dense induction zone (12) and at least one first corresponding zone (13), the at least one first sparse induction zone (11) and the at least one first dense induction zone (12) corresponding to the at least one second corresponding zone (24) and overlapping therewith, the at least one second sparse induction zone (21) and the at least one second dense induction zone (22) corresponding to the at least one first corresponding zone (13) and overlapping therewith.

4. The induction pad of claim 2 or 3, wherein the at least one second dense induction zone (22) includes a plurality of induction members (23) arranged denser than that in the at least one second sparse induction zone (21).

5. The induction pad of claim 4, wherein the plurality of induction members (23a) are formed in a lattice structure.

6. The induction pad of claim 5, wherein each of the plurality of induction members (23, 23a) is electrically connected to a detection unit (80).

7. The induction pad of one of claims 1 to 6 further including a pliable layer (40) connecting to and overlapping with the second induction layer (20), wherein the second induction layer (20) is interposed between the pliable layer (40) and the spacer layer (30).

8. The induction pad of claim 7 further including a rigid layer (60) connecting to and overlapping with the first induction layer (10), wherein the first induction layer (10) is interposed between the rigid layer (60) and the spacer layer (30).

9. The induction pad of claim 7 or 8 further including a waterproof and air permeable layer (50) connecting to and overlapping with the pliable layer (40), wherein the pliable layer (40) is interposed between the waterproof and air permeable layer (50) and the second induction layer (20).

10. The induction pad of one of claims 1 to 9, wherein the at least one high pressure spacer zone (31) includes a plurality of spacers (33) arranged denser than that in the at least one low pressure spacer zone (32).

11. The induction pad of one of claims 1 to 9, wherein the at least one high pressure spacer zone (31) includes a plurality of spacers (33a) formed thicker than that in the at least one low pressure spacer zone (32).

12. The induction pad of one of claims 1 to 11, wherein the second induction layer (20) includes a plurality of apertures (28) for air ventilation.

13. The induction pad of one of claims 1 to 12, wherein the spacer layer (30) is a sheet, the at least one high pressure spacer zone (31) and the at least one low pressure spacer zone (32) distributing a plurality of holes (34).

14. The induction pad of one of claims 1 to 13, wherein the first induction layer (10), the second induction layer (20) and the spacer layer (30) are made from flexible material.
